# EUROPEAN PATENT SPECIFICATION

(11) **EP 0 389 629 B1**
(45) Date of publication and mention of the grant of the patent: **11.05.1994**
(21) Application number: 89907293.8
(22) Date of filing: 22.06.1989
(51) Int. Cl.: A61L 25/00, A61K 6/06

(54) **MEDICAL AND DENTAL CURABLE MATERIAL**
ÄRZTLICHES UND ZAHNÄRZTLICHES VERNETZBARES MATERIAL
SUBSTANCE DURCISSANTE POUR UTILISATIONS DENTAIRES ET MEDICALES

(30) Priority: 10.08.1988 JP 200617/88
(43) Date of publication of application: 03.10.1990
(60) Divisional application: 92122177.6
(73) Proprietor: NITTA GELATIN INC., Osaka-shi Osaka 541 (JP)
(72) Inventor: OONISHI, Hironobu, Osaka-shi Osaka 545 (JP); SUGIHARA, Fumihito, Osaka 596 (JP); ISHII, Takashi Room 302 Apple-Tennoji, Osaka 543 (JP); SUZUKI, Kaneo, Kashihara-shi Nara 634 (JP); HATA, Seiko, Minamikawachi-gun Osaka 587 (JP); TAKANO, Toshikazu, Ikoma-gun Nara 636 (JP)
(74) Representative: Pacitti, Pierpaolo A.M.E.
(86) International application number: PCT/JP89/00620
(87) International publication number: WO 90/01341

(56) References cited:
- JP-A-58 183 607
- JP-A-63 153 070
- PATENT ABSTRACTS OF JAPAN, vol. 9, no. 160 (C-289), 4th July 1985; & JP-A-60 036 404 (MIRAI KAGAKU KENKYUSHO) 25-02-1985

## Description

### [ Technical Field ]

This invention relates to hardening material for medical and dental use (hereinafter simply referred to as "hardening material") which is used for bone cement, cement for dental use, and root canal sealing material.

### [ Background Art ]

In recent years cement for dental use typically comprises a powder of hydroxyapatite (hereinafter referred to as "HAp") and α-tricalcium phosphate (α-Ca3( P04 )z: hereinafter referred to as "α-TCP") and a setting solution, being an aqueous solution of polyacrylic acid. A hardened product is made by mixing and kneading the powder with the setting solution. However, a residue of unreacted polyacrylic acid sometimes remains after hardening is complete. Accordingly, there exists a problem that the body to which the material is applied may suffer damage due to subsequent elusion of the acid.

Other known cements for dental use and root canal sealing materials, include cements and root canal sealers in a series of zinc oxide eugenol in which eugenol is mixed with a setting liquid to provide a pain-killing effect. However, cell toxicity has been reported with eugenol. Also, a composite resin used for recovery of a tooth crown part is disturbed in polymerization with eugenol. Therefore, there exist a number of problems associated with materials for dental use in the eugenol series.

To date, there have also been marketed certain types of bone cement in which polymer materials such as polymethylmethacrylate (PMMA) and methylmethacrylate (MMA) are used. However, the following three problems occur with these types of bone cement in which such polymer materials are used. Firstly, bone tissue in a host site, which is subjected to plugging up, does not directly combine with the bone cement. Accordingly, when the bone cement is plugged up in a living body for a long period, problems arise such as loosening due to interposition of a fibril tissue. Secondly, since temperatures in the range 90 - 100 °C are generated during hardening of such materials, surrounding cells may be damaged or destroyed. Thirdly, there exists a problem that elusion of a residual monomer or oligomer which has not reacted during hardening may have a damaging effect on the bone.

On the other hand, there have been proposed a number of hardening materials which include α-TCP powder or tetracalcium phosphate powder (Ca₄(P0₄)₂ 0 hereinafter referred to as "4CP"). These substances are analogous to HAp, which is a main inorganic component of hard body tissue. The powders are used with a setting liquid composed of a solution of some type of organic acid. For example, in JP-A-60-36404 there is described a material comprising α-TCP powder and a setting solution of 1 M tannic acid. In JP-A-62-12705 there is described a material comprising α-TCP powder and a 30 - 60 % (w/w) aqueous solution of citric acid. Also, in JP-A-62-83348 there is described a material comprising α-TCP powder and a 45% (w/w) aqueous solution of hydroxysuccinic acid. The α-TCP and 4-CP are of high chemical reactivity and can be converted into HAp under conditions similar to those in the body or mouth.

The hardening materials described in the above publications have such properties as to cause almost no damage to a body, have the capability to form a hardened product analogous to hard body tissue, and are able to combine with hard tissue. A hardening material including α-TCP or 4CP as its powder component together with a solution of an organic acid as its setting liquid component is very useful for medical and dental use, so that its practical use is highly acceptable.

Such hardening materials and their hardened products cause no damage to the body, but if the ratio between the calcium phosphate powder and the setting solution (hereinafter simply referred to as "powder: liquid ratio") becomes too great, the time for hardening becomes extremely short, so that the material is of limited practical use.

Hardening materials are classified, for example, in the undermentioned major two classes (a) sealing agents, and (b) cements.
(a) Sealing agents are used in circumstances where only relatively low forces are encountered. The agent is used for plugging up and blocking up a lost gap or as a firmly fixing supporter, and the time for hardening of the agent is very long. Sealing agents do not exhibit high mechanical strength, especially in resisting force against crushing, and it is preferred if the agent slowly releases a substance which has a pain-killing effect. Such materials may be used as bone sealers or root canal sealers etc.
(b) Cements are used in circumstances where relatively high forces are encountered. The agent is used for plugging up and blocking up a lost gap or for the purpose of bonding hard body tissues to one another, bonding hard body tissue to another material, or bonding other materials to one another. The time for hardening following mixing should be relatively short, and, after hardening, the agent exhibits relatively high mechanical strength especially in resisting force against crushing. It is preferred that cement materials should chemically bond with hard body tissues. Examples of such materials are bone cement, cement for dental use, and adhesive agents for dental use.

In the light of the foregoing, it is an object of the present invention to provide a hardening material which can be applied for use as a sealing agent as described at (a) above and/or as a cement as described at (b) above, which undergoes hardening at or below room temperature or body temperature, which has no injurious effects on the body, which forms a hardened product analogous to a hard body tissue and is capable of bonding with hard body tissue, and which has a hardening time which is freely controllable without reducing its working efficiency as regards mixing and kneading.

### [Disclosure of Invention]

The hardening material relating to the present invention is characterized in that calcium phosphate powder containing at least either one of α-TCP and 4CP is included as an essential component and, at least one compound selected from collagen and collagen derivatives and one kind or more of organic acid are used as a hardening adjuster.

Hereinafter, the present invention is explained in detail.

The hardening materials relating to the present invention is comprise a combination of, at least, calcium phosphate powder and a setting liquid.

At least either one of α-TCP and 4CP forms a part or the whole of the calcium phosphate powder. Any residual part of the powder comprises HAp, apatite carbonate, -tricalcium phosphate (hereinafter referred to as " -TCP"), and calcium hydrogen phosphate dihydrate etc. In a preferred embodiment, the calcium phosphate powder comprises 4CP in the range 10 - 100 % (w/w), α-TCP in the range 0 - 90 % (w/w), and HAp in the range 0 - 30 % (w/w).

If 4CP comprises less than 10 % (w/w) of the calcium phosphate powder, then it is possible that, after mixing and kneading, the physical strength of the hardened product will be extremely low. If HAp comprises more than 30 % (w/w) of the calcium phosphate powder, the time for hardening becomes excessively short such that there may be insufficient time for adequate mixing and kneading. 4CP is more reactive than α-TCP, leading to a short pot life and difficulties in handling. Accordingly, reactivity may be reduced by adding α-TCP.

In a further preferred embodiment the calcium phosphate powder comprises 60 - 100 % (w/w) α-TCP and 0- 30 % (w/w) HAp.

In this case, if α-TCP constitutes less than 60 % (w/w) of the calcium phosphate powder, then it is possible that, after mixing and kneading, the physical strength of the hardened product will be extremely low. If the calcium phosphate other than α-TCP and HAp comprises more than 10 % (w/w) of the powder, the hardening may be insufficient, or the time for hardening may become excessively short such that there is insufficient time for adequate mixing and kneading. Furthermore, components other than 4CP, α-TCP, and HAp are preferred to comprise 40 % (w/w) or less of the calcium phosphate powder. If such components exceed 40% (w/w), the physical strength of the mixed and kneaded, hardened product may become extremely low.

Also, any powder other than said calcium phosphate, which does not disturb the reaction, can be included to make up to 30 % (w/w) of the total powder agent. Suitable powders other than calcium phosphate include, for example, a barium salt, a bismuth salt, a zinc salt, and oxides thereof, (which provide enhanced X-ray contrast), or a dye such as -carotin, a pigment such as titanium dioxide, or a fluoride such as calcium difluoride.

Provided that they do not interfere with the reaction and do not cause undesirable effects on physical properties, all kinds of powder may be substituted for purposes other than those noted above.

Preferably, the powder has an average particle diameter in the range 1 - 25 µm. If the average particle diameter of the powder is less than 1 µm, the physical strength of the hardened product may increase, but the time for hardening time becomes short. If it is more than 25 µm, especially in the case where it is used for. cement for dental use, there may be a problem that the film thickness of the hardened product does not become 30 µm or less.

4CP is obtained, for example, by baking followed by pulverizing a composition of -ca₂p₂o₇ and C₃C0₃ in a 1 : 2 molar ratio at a temperature of 1300 °C or more, but 4CP obtained by other methods can be used. The α-TCP is obtained, for example, by baking followed by pulverizing a composition of -ca₂p₂o₇ and CₐC0₃ in an equal molar ratio at a temperature of 1200 °C or more, but also α-TCP obtained by other methods can also be used. The HAp etc. may be calcium phosphate originated from a living body as well as powdered bone or may be a synthetic HAp, apatite carbonate, or -TCP etc. obtainable by methods known in the art. Calcium phosphate of these kinds has no injurious effects upon the body.

The organic acid used may be an acid of one kind alone or a mixture of two or more kinds of acid, selected from a group of organic acids relating to the living body such as, for example, citric acid, hydroxysuccinic acid, malonic acid, glyceric acid, and glutaric acid. These organic acids give a hardened product of hard quality by being mixed and kneaded with the calcium phosphate powder. Concentration of the organic acid in an organic solution is not especially defined, but a range of 0.1- 90 % (w/w) is preferred. A range of 0.1-70% (w/w) is more preferred. Below these ranges, after mixing and kneading, the hardened product has reduced physical strength and may sometimes collapse in an aqueous solution. Above these ranges, crystals sometimes separate from the setting solution before mixing and kneading.

In the present invention, collagen and/ or collagen derivatives (hereinafter simply referred to as "collagen") are used as powder or in a melted state. This choice is properly carried out according to a procedure. In any case, when a powder component and a liquid component are mixed and kneaded, it is preferred for the collagen that it is dissolved prior to fibrillation taking place during hardening. If the collagen has already converted into fibrils when it is mixed and kneaded, there is a possibility that the fibrils may separate.

In the case where collagen is used in a melted state, collagen can be used by dissolving it into the setting solution or it can be used by preparing a collagen solution separate from the setting solution. When collagen is dissolved, an aqueous solution is prepared by dissolving it into water or into a setting solution of dilute concentration. Where collagen is used in a powder state, it may be mixed with the calcium phosphate powder or kept separate from the calcium phosphate powder.

The proportion of collagen used is preferably in the range 0.02 - 100 parts by weight against 100 parts by weight of the calcium phosphate powder. If the proportion for use of collagen deviates from this range, it is possible that chemical bonding at an interface between a coagulated, hardened product and hard body tissue will be weakened or that mixing and kneading will become difficult.

The collagen used may be of one kind, two kinds or more selected from: collagen treated with alkali, collagen made soluble with neutral salts, collagen made soluble with enzyme, and derivatives of these collagens.

Certain kinds of collagen generally undergo fibrillation within a very short time under physiological conditions (for example, pH 7.0 - 7.4, temperature of 36 - 37 °C, salt concentration of 0.14 M). Accordingly, if such a kind of collagen is used as a hardening adjuster, collagen which has converted into fibrils coagulates and sometimes separates from a calcium phosphate-coagulated product. If this separation takes place, a complex derived from a chemical binding of HAp with collagen cannot be obtained. Thus, to obtain this complex, collagen which undergoes fibrillation relatively slowly is preferred for use. However, if the collagen species have this character, they are not limited to the type I collagen, and the type II, III and IV collagens also can be used. Said very short time means 8 minutes or more, preferably about 10 minutes.

Collagens which do not undergo fibrillation under physiological conditions include, for example, decomposed gelatin (water-soluble gelatin or gelatin 21, products of Nitta Gelatin Inc.), type IV collagen (type IV collagen produced by Collagen Corporation), collagen made soluble with neutral salts (type I collagen), collagen treated with an alkali (type I collagen), succinated collagen (type I collagen), and methylated collagen (type I collagen). Collagens which do undergo fibrillation under physiological conditions within 8 minutes include, for example, Cellmatrix type I-A produced by Nitta Gelatin Inc. and Cellgen I-AC produced by Kouken Co., LTD. (both are collagens soluble in an acid (type I collagen)) and so on.

Collagens which undergo fibrillation with a time longer than 8 minutes include, for example, atelocollagen (type I collagen: Cellmatrix LA produced by Nitta Gelatin Inc, Cellgen produced by Kouken Co., LTD., Vitrogen-100 produced by Collagen Corporation and so on.), collagen soluble in an enzyme (type I collagen : Cellmatrix type I-P produced by Nitta Gelatin Inc. and so on.), type II collagen (Cellmatrix Type II produced by Nitta Gelatin Inc. and so on.), type II collagen (Cellmatrix type II produced by Nitta Gelatin Inc. and so on.), type IV collagen (Cellmatrix type IV produced by Nitta Gelatin Inc. and so on.). In this invention, these kinds of collagens can be used with proper selection.

Where collagen is used in this invention, both fibrillation of the collagen and coagulation and hardening of the calcium phosphate proceed in parallel or almost in parallel, and a hardened product derived from coalescence of collagen fibrils and a calcium phosphate hardened product into one body can be obtained. With these, the hardened product obtained makes a sufficient chemical bond with hard body tissue.

Atelocollagen is the most preferred collagen for use. Atelocollagen is, for example, collagen in which a part or a whole of teropeptide at a terminal end of the molecule is removed by treatment with an enzyme and it has no injurious effect upon the body. Collagen may be used by dissolving it in a setting solution, as a solution independent of the setting solution, or in a powder state. Although the collagen concentration in a collagen solution is not especially limited, it is preferred to be in a range of 0. 01 - 35 % (w/w). The more preferred concentration is in a range of 0.05 - 35 % (w/w).

Below these ranges, the delaying effect on hardening by collagen may not be displayed. Above these ranges, collagen may decompose in a solution of an organic acid before mixing, or the viscosity of the solution may be raised excessively. Where collagen is used in a powder state, powder having the forementioned average particle diameter is favoured for the reasons discussed. Suitable collagen derivatives include for example, gelatin, decomposed gelatin (or polypeptide), succinate collagen, and methylated collagen.

In the present invention, the progress of the hardening reaction of calcium phosphate powder is adjusted by means of the combination of collagen, collagen derivatives, and organic acids being used. In this way, operational efficiency for kneading is improved, the ratio of powder to liquid can be raised, and a hardened product of higher strength can be obtained. These may also be used in applications requiring a relatively long time for sealing, for example, in root canal sealer for filling a cavity in a tooth root canal.

The hardening adjustment provided by the present invention is discussed by reference to (I) and (II) below.
( I ) A given compound used in combination with either one of α-TCP and 4CP and undergoes hardening over a much longer time (for example, one hour or more), compared with prior setting solutions composed of only an organic acid, and does not lower operational efficiency for kneading.
( II ) Where the hardening is carried out by using at least one of either α-TCP and 4CP, water, and one or more additional compound, if hardening is carried out by using a further one additional compound (that is, in total, two or more kinds of compounds) the time for hardening can be controlled without lowering operational efficiency for kneading. In this case, said two or more kinds of compounds.

The hardening adjustment discussed at (II) will now be explained in more detail. When said additional one kind of compound is added, the compound which is replaced as a result may be α-TCP, 4CP, water, or other components which constitute a hardening material. When said additional one kind of compound replaces any other component constituting a hardening material, it is required that the time for hardening can be controlled, after replacement more than before replacement, without lowering the operation efficiency for kneading.

In the case that said hardening adjuster provides a hardening material, an essential reason is as follows.

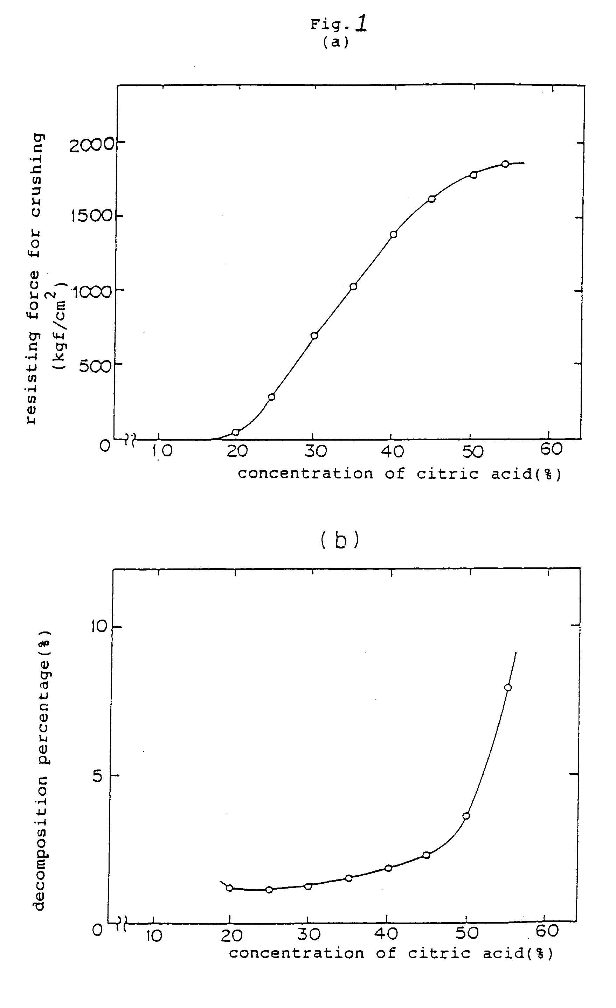

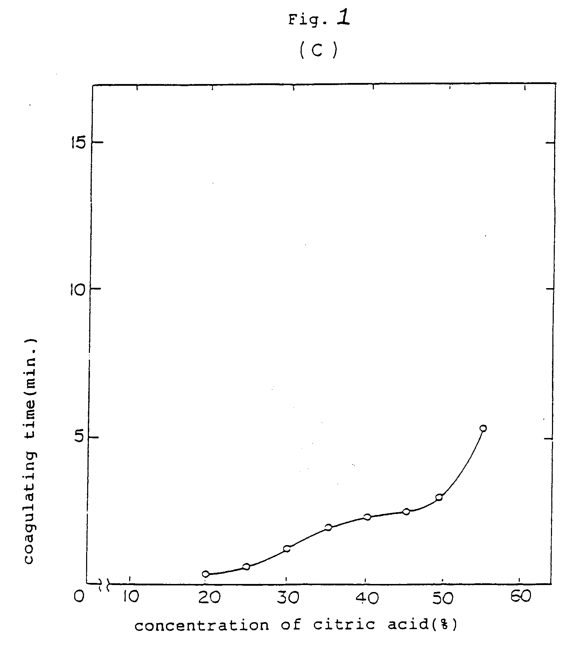

When there is a hardening material composed of α-TCP and/ or 4CP, water, and one kind of organic acid (for example, an aqueous solution composed of 100 % of α-TCP and an aqueous 45 % solution of citric acid), two methods may be used to delay the hardening without use of a hardening adjuster. The first method, as seen in Fig. 1 (c), is a method in which the concentration of the solution is raised (that is due to replacement of water by an organic acid), but this method requires increasing force for kneading as concentration of the organic acid increases, so that operational efficiency is adversely affected. The second method is a method in which the ratio of powder to liquid is lowered (the liquid proportion is raised due to replacement of calcium phosphate powder with water and an organic acid ). However this method shows decreasing strength as the ratio of powder to liquid becomes lower so that the physical properties of the hardened material are adversely affected; for example, by an increase of decomposition percentage. The above described hardening adjuster serves to delay hardening while compensating for these adverse effects (lowering of operation efficiency during kneading and of physical properties after hardening).

Powder agent and/or setting liquid in the hardening materials of the present invention may be used with the addition, if necessary, of any one or more materials selected from: polysaccarides such as arginic acid, carrageenan, pectin, xanthane gum, locustbean gum, and jellan gum, which converts into a gel by a calcium ion, and mucopolysaccharide, chitin, and chitosan. Also, in order to add viscosity during operation, so as to improve operation efficiency, polyalkylene glycol, polyethylene glycol, polyvinylalcohol, polyvinylpyrrolidone, dextran and so on may be added to provide an adhering agent for said powder agent and/or setting liquid, in a degree such that they do not participate in reaction and do not adversely affect the physical properties of the hardened material.

The hardening materials in this invention are able to undergo hardening by mixing and kneading at about room temperature or the temperature of a living body and thus, there is no problem of cell death by generated reaction heat.

The hardening materials in the present invention, for example, are as follows.
**1** A system composed of combination of a calcium phosphate powder, an organic acid, and collagen.

In this system also, an organic acid and collagen are hardening adjusters. Collagen may be used by preparing a solution independent of an organic acid solution, by dissolving it into an organic acid solution, or in a powder state.

Although the proportions of materials in system 1 are not especially limited, a range of 5 - 70 parts by weight of an organic acid and a range of 0.01- 30 parts by weight of collagen against 30 - 80 parts by weight of calcium phosphate powder are preferred. If the organic acid is below this range, hardening may be insufficient. Above this range, the delaying effect of collagen on hardening may not be obtained. If collagen is below this range, the strength of the hardened product is sometimes not enhanced. Above this range, kneading under room temperature is not sufficiently possible.

A reaction mechanism of materials in said system 1 is considered, on the basis of analysis results from X-ray powder diffraction, infrared absorption spectra, and scanning electron microscope, for example, as analogous to a collagen-calcified model of a living body hard tissue, as follows. When calcium phosphate powder, an organic acid, and collagen are mixed and kneaded at room temperature or around living body temperature, a chelate bond is formed between calcium atoms of 4CP and α-TCP in the powder and the carboxyl groups in the organic acid and thus, a neutralization reaction progresses. On the other hand, collagen converts into fibrils and the chelated product coheres with the collagen fibrils. In the presence of water and at room temperature or around living body temperature, the chelated products, existing on a surface of the hardened product and on a surface of the pores, and the unreacted 4CP and α-TCP form OCP and ACP by undergoing a hydration reaction. Subsequently, the OCP and ACP convert into HAp which crystallizes into collagen fibrils, and thereby the hardening progresses.

When the materials in said system 1 are mixed and kneaded, the progress of the hardening becomes slower than in the case of where collagen are not used. For example, at room temperature or around living body temperature the hardening is completed in 5 - 60 minutes after the initiation of kneading and a hardened product of hard quality is obtained. Because of this, the ratio of calcium phosphate powder to organic acid can be raised, so that the strength of the hardened product can be increased. In particular, if collagen is used, compression strength is increased without raising the ratio of calcium phosphate powder to organic acid. In addition, after hardening is completed, the compression strength increases with time passage and elasticity is enriched. The materials in said system 1 can be used as sealing, bonding, or dental prosthesis materials and for the hard tissue of a living body; for example, bone cement and cement for dental use and so on.

When a hardening product at an initial stage obtained from mixing and kneading of the materials in said system 1 is immersed in a physiological PBS (phosphate-buffered saline) at 37 °C, the resisting force for crushing increases with time passage.

When the materials in said system 1 are converted into bone cement and then buried in bone of a living body, the materials form a bone-like structure and coalesce into one body with the bone tissue, since the cement is active for the living body. In the case where α-TCP is used, since α-TCP is biodegradable it can be gradually substituted by new bone during a period from of 6 months to 1 year. The material is substituted by a bone tissue with the passage of time. Thus, the material coalesces into one body with already existing hard tissue.

Besides, all the materials in said system 1 may contain any material other than the above-described materials as far as the desired properties of present invention are not compromised.

Also, use is not limited to the particular examples disclosed herein.

All the materials in said system 1 can control the time period for hardening without lowering operation efficiency for kneading, by virtue of the setting agents used therein.

Hereinafter, the process whereby the present inventors found the forementioned means for solving the problems addressed by the invention is explained in detail. The present inventors, in addressing said problems, studied the reasons why calcium phospate materials previously proposed have not been used in practice. As a result, it was found that the hitherto known hardening materials are not satisfactory in all the undermentioned respects (1) - (3) and are defective in at least one these respects.
( 1 ) Strength of the hardened product should be high.
( 2 ) Under physiological conditions decomposition character should be low.
( 3 ) During mixing and kneading, the hardening process should proceed sufficiently slowly and the efficiency for operation should be superior.

In order to obtain materials which are satisfactory in all of these respects and which show superior affinity for the living body hard tissue, the inventors carried out studies from the basis that, rather than using a calcium phosphate besides α-TCP with the addition of a component other than the organic acid to the setting solution, a superior idea would be to use an organic acid which is known, according to previous research, to have no injurious effects upon the body and to determine its most suitable concentration range.

Organic acids for use in the hardening materials which are known to be likely to be suitable for use include:-monocarboxylic acids, dicarboxylic acids, and tricarboxylic acids. In particular, the dicarboxylic acids and the tricarboxylic acids in the Krebs cycle are superior complex-forming reagents for calcium and a high liklihood of suitability for use is expected. For almost all the monocarboxylic acids and for the dicarboxylic acids, since the chelate-forming force is weak, the decomposition percentage often rises dramatically after hardening is complete (for example, pyruvic acid, glyceric acid, and lactic acid, and also, as a dicarboxylic acid, maleic acid). Also, due to said weak chelate-forming force, some acids result in a long hardening time (for example, in the cases of lactic acid and glucuronic acid ). Due also to said weak chelate-forming force, there are acids with which calcium salts are formed within a short time and, as a result, the hardening time becomes extremely short (for example, of the monocarboxylic acids, pyruvic acid, and of the dicarboxylic acids, tartaric acid, oxalic acid, and glycolic acid ). Although the tricarboxylic acids have a relatively strong chelate-forming force and are mostly expected to show superior physical properties after hardening (in strength and decomposition percentage etc.), the majority of such acids show poor solubility in water and are hard to obtain in suitable concentration (for example, aconitic acid, oxaloacetic acid, and oxalosuccinic acid etc.). Also, among the dicarboxylic acids, some acids show poor solubility (for example, succinic acid and fumaric acid etc.). From consideration of these, those acids of high solubility in water in a group of the di- or tricarboxylic acids can be expected to be of practical use.

Accordingly, acids having no injurious effects upon the body selected from the great number of organic acids believed likely to be usable as hardening materials were each used in a form of an independent aqueous solution as a setting solution, and were examined as to whether or not a concentration range existed which is satisfactory in each of the respects ( 1 ) - ( 3 ) described above. At first, the solubilities of the organic acids in water were examined and, as a result, high solubility was indicated with a circle and poor solubility with a cross as in table 1. The acids of poor solubility were excluded from the examination list and the acids of high solubility were further examined. The variation of resisting force for crushing (kg f/cm2 ) when the citric acid concentration is varied is shown in Fig. 1 (a), the variation of decomposition percentage (%) with the above variation in Fig. 1 (b), and the variation of coagulating time (min.) with the above in Fig. 1 (c). On the basis of these graphs, said concentration range was examined. Other organic acids were also similarly examined.

In table 1, the strength for crushing corresponds to said (1) above, the decomposition percentage to (2) and the coagulating time to (3) respecitvely. These capacities were examined on the basis of JIS T6602 under the conditions that the ratio between powder and liquid was 2.5 and the mixing and kneading were performed by hand. Results are presented for each of the properties (1) - (3) by the presence of a circle if they are of practically useful standard, of a cross if they are definitely of an unpractical standard, and of a triangle if they are of a standard somewhat inferior to the practical standard. Also, a circle indcates if a concentration range exists in which all three properties (1)-(3) are satisfied to a practical standard at the same time, and a cross indicates that no such range exists.

As seen in Table 1, none of the organic acids so far proposed have a concentration range which is satisfactory in all three respects (1) - (3). However, citric acid, malic acid, and malonic acid are in outline satisfactory for (1) - (3) (that is, there are no "cross" marks in any of the three individual categories (1)-(3)). The other organic acids are unsatisfactory in at least one of these categories. Also, the various organic acids differ with regard to said properties (1) - (3). These differences are believed to arise from decarlification ability, rate of chelate-forming reaction and binding force with Ca²⁺, and the molecular weight of the organic acids, pH and stability with Ca²⁺ of the mixed and kneaded products, and difference of varying percentage in the hardening density.

On the other hand, if the concentration of an organic acid in a setting solution is high, the resisting force for crushing becomes high, the coagulating time tends to be long, the decomposition percentage tends to be high, a considerable amount of force may be needed for the kneading operation, and the sealing of a small gap in a living body may become difficult. Futhermore, if the concentration of an acid is high, unreacted acid may undergo elution, giveing an impetus to a living body and may cause an imflammatory reaction. On the contrary, if the concentration of an organic acid is low, hardening may take place very quickly after mixing, and use tends to become difficult. From consideration of these factors, it is hoped that the concentration of the organic acid is about 35 - 50 %.

For the hardening materials relating to the present invention, as mentioned above, a main material is calcium powder in which at least either one component of 4CP and α-TCP is an essential component and the material according to the invention is used as a hardening adjuster, so that hardening proceeds at room temperature or around living body temperature and the hardening time can be elongated with virtually no decrease in the operational efficiency for mixing and kneading and so that there is injurious effect upon the body.

Therefore, the hardening materials relating to the present invention can be utilized for uses wherein a long hardening time is required, and for uses wherein a hardened product of high strength is needed,by raising the ratio of calcium phosphate powder to the setting agent.

The hardening materials in this invention are not limited to those which contain only the essential components mentioned above, and other materials may be combined to an extent which does not compromise the attainment of the objects of the invention.

### (Brief Description of the Drawings)

Fig. 1 (reflecting the state of the art) shows the concentration when a single organic acid is used alone, wherein Fig. 1 ( a ) shows resisting force for crushing, Fig. 1 ( b ) shows decomposition percentage, and Fig. 1 ( c ) shows coagulating time.

### (Best Mode for Carrying out the Invention)

Hereinafter, examples of the present invention are presented with examples for comparison, but the invention is not limited to said examples.

### Examples 1 - 7 and examples for comparison 1 - 4.

Solutions containing collagen, and organic acids were prepared in the concentrations shown in tables 2 and 3. The solutions were mixed with calcium phosphate powders, the compositions of which and the ratios of powder to liquid are also shown in tables 2 and 3, and kneaded by hand for about one minute. The undermentioned measurements were carried out by using the kneaded mud and the results obtained are shown in tables 2 and 3. Powder having an average particle diameter of 7 µm was used and atelocollagen (Cellmatrix LA produced from Nitta Gelatin Inc.) was used as collagen. Besides, in the undermentioned measurements, all were carried out according to ADAS No. 61 under conditions in which the temperature was 23 +/- 2°C and relative humidity of 50 +/- 10 %.

### ( a ) Measurements of time for hardening at an initial stage

Each sample of kneaded mud was poured into a metal mold of a cylinder shape made of stainless steel, which had an inner diameter of 10 mm and a height of 5 mm, and which was placed on a glass plate having length, width, and thickness all of 15 mm. The surface was made even and, one minute after the kneading finished, the mud was transferred into a high temperature vessel at a temperature of 37 +/- 1 °C and humidity of 100 % to prepare a piece for examination. The time at which a bikkar needle having a weight of 2.94 N (300 g) and a section area of 1 mm² failed to leave a needle trace when when dropped on to a surface of the piece being examined was assigned as the hardening time at an initial stage, calculating from the beginning of kneading. The hardening time at the initial stage was derived by taking an average of the values obtained from three sets of time measurements taken at 15 second intervals.

### ( b ) Measurements of resisting force for crushing

Each sample of kneaded mud was poured into a metal mold of a cylinder shape made of stainless steel, which has an inner diameter of 6 mm and a height of 12 mm. Both ends were pinched between thick glass plates and then pressurized. 2.5 minutes after kneading was initiated, the mud was transferred, maintaining the pressurisation, into a high temperature vessel which was kept at a temperature of 37 +/- 1 °C and relative humidity of 100 %. After one hour the hardened product was removed from the metal mold, immersed in distilled water at 37 +/- 1 °C, and removed from the distilled water after a period of 24 hours from the initiation of kneading, for examination. The sample for examination was subjected to mesurements of resisting force for crushing using a Shimazu Autograph AG-2000 A. At a crosshead speed of 1 mm per minute, the measurements were carried out on six samples and the value measured was obtained by averaging those numeral values which remained after removal of values showing 15 % or less of the total average value. In cases where there were two or more values showing 15 % or less of the total average value, the measurements were carried out again.

As seen in tables 2 and 3, comparing the examples 1 - 4 with the examples for comparison 1 and 2, and the examples 5 - 7 with the examples for comparison 3 and 4, the examples showed longer hardening time at the initial stage. In addition, since a setting solution containing one or more kinds of organic acids was used for the examples for comparison 1 - 4, in order to delay the hardening time, the concentration of the setting solution may be increased or the ratio between powder and liquid may be reduced. However, if the concentration of the setting solution was increased, greater force was required for kneading, and if the ratio between powder and liquid was reduced, the resisting force for crushing at an initial stage tended to be lower. Even though, as shown in examples 4 and 7, the ratio between powder and liquid was increased and the resisting force for crushing of the hardened product was enhanced, the hardening time at an initial stage was not increased to an extent that caused any practical problem.

Also, in the cases where collagen was used (examples 1 - 7), the resisting force for crushing at an initial stage clearly increased.

When each of the materials in examples 1 - 7 and the examples for comparison 1- 4 were immersed in PBS, in the cases where collagen was used it was seen that even after hardening at the initial stage, the resisting force for crushing continued to increase with the passage of time.

Further, each material in examples 1 - 7 was hardened at an initial stage to a form a cylindrical piece having a diameter of 6 mm and a length of 12 mm, which was buried in a defective part of the femur of a dog, removed after standing, respectively, for 2, 4, and 6 weeks, and evaluated by tissue observation at a surface conjugated with bone tissue and by a pushing-out method so as to observe conjugation force with the bone. As a result, in each material of examples 1 - 7 no inflammatory reaction of such a kind was found and a direct conjugation with the bone had already progressed. When the materials of examples 1 - 7 were stood for 4 and 6 weeks after transplantation, there existed bone cells in an interface with the bone tissue. Especially, a number of bone cells existed in the circumference of the interface and the rigidly conjugating force with the bone increased rapidly.

### Example 8

A hardening material was prepared composed of a powder agent, comprising 80 % of 4CP and 20 % of α-TCP, and a setting solution (a liquid agent) made by dissolving into water malic acid, citric acid, and acid-soluble collagen (Cellmatrix type I-A produced by Nitta Gelatin Inc, which converts into fibrils within 8 minutes under physiological conditions) in respective proportions of 40, 10, and 0.5 %. In this example the malic acid, citric acid, and collagen are hardening adjusters.

### Example 9

A hardening material was prepared composed of a powder agent, comprising 80 % of 4CP and 20 % of α-TCP, and a setting solution (a liquid agent) made by dissolving into water malic acid, citric acid, the type II collagen (Cellmatrix type II produced by Nitta Gelatin Inc., which does not convert into fibrils under physiological conditions), and decomposed gelatin (water-soluble gelatin produced by Nitta Gelatin Inc., which does not convert into fibrils under physiological conditions) in respective proportions of 40, 10, 0.5, and 1 %. In this example the malic acid, citric acid, type II collagen, and decomposed gelatin are hardening adjusters.

Each hardening material in examples 8 and 9 was mixed in the ratio of powder to liquid shown in table 4 and kneaded as in example 1. Subsequently, the time for hardening at the initial stage and the resisting force for crushing were examined as carried out in example 1. Results are presented in table 4.

The properties of interest did not decrease and, as shown in table 4, the time for hardening at the initial stage was adjusted (refer to the examples for comparison 1 and 2 in table 2).

Examples in the case where the hardening materials of the present invention are used for a root canal sealer are shown hereinafter with examples for comparison.

### Example 10

A hardening material was prepared composed of a powder agent, comprising 98 % of 4CP and 2 % of carboxymethylchitin, and a setting solution (a liquid agent) prepared by dissolving into water atelocollagen (Cellmatrix LA, produced by Nitta Gelatin Inc.), malic acid, and citric acid in respective proportions of 0.5 %, 18 %, and 4.5 %.

### Example 11

A hardening material was prepared composed of a powder agent, comprising 95 % of 4CP and 5 % of atelocollagen (Cellmatrix LA, produced by Nitta Gelatin Inc.), and a setting solution (a liquid agent) prepared by dissolving into water aluginic acid, malic acid, and citric acid in repective proportions of 0.5 %, 18 %, and 4.5 %.

### Example 12

A hardening material was prepared composed of a powder agent of 100 % of 4CP and a setting solution (a liquid agent) prepared by dissolving into water atelocollagen (Cellmatrix LA, produced by Nitta Gelatin Inc.), xanthan gum, malic acid, and citric acid in respective proportions of 0.3 %, 0.3 %, 15 %, and 3.7 %.

### Example for comparison 5

A root canal sealer was prepared composed of a powder agent, comprising 20 % of 4CP, 20 % of MgO, 20 % of rosin, and 40 % of bismuth bicarbonate, and a solvent composed of 100 % of oleic acid.

### Example for comparison 6

A root canal sealer was prepared composed of a powder agent, comprising 43 % of 4CP, 20 % of MgO, 30 % of bismuth bicarbonate, and 0.7 % of Ca(OH)₂, and a solvent composed of 100 % of euginol.

### Example for comparison 7

A commercially available root canal sealer, from Showa Yakuhin Kagaku Kogyo Co., Ltd. (trade name Kyanarusu), was used.

For the materials in the examples 10 - 12 and the examples for comparison 5 - 7, the flow (ie. the degree of flowing under applied pressure), time for hardening, solubility, degree of decomposition
(referred to as "decomposition percentage"), and resisting force for crushing were determined according to the ISO standards (International Organization for Standardization) 6876-1986 (E). Furthermore, cement or a root canal sealer was applied under pressure in a location from which the molar dental pulp of a grown dog had been extracted, and then stood for 3 months. Tooth extraction was followed by setting and then a slice for polishing, which did not decalcificate, was subjected to a pathological observation with haematoxyline-eosine (referred to as "H ·E") coloring.
1 ) Flow ( degree of flowing under applied pressure);
   A root canal sealer was kneaded, and 0.075 ml thereof placed on a glass plate. 3 minutes after kneading was initiated, a load of 2.5 kg was imposed. The flow was determined from the resulting increase in diameter of the kneaded mud. According to the ISO standards, the value is defined as 20 mm or more.
2 ) Time for hardening;
   A ring having a diameter of 10 mm and a height of 2 mm was filled with kneaded mud. The time elapsed from 2 minutes after kneading being initiated when a Gilmore needle having a load of 100 g and a diameter of 2 mm fails to produce a mark in the mud under conditions of ordinary temperature of 37°C and relative humidity of 95 % or more, is defined as the time for hardening.
3 ) Solubility and decomposition degree (decomposition percentage);
   A ring having a diameter of 20 mm and a thickness of about 1.5 mm was filed with kneaded mud. Under conditions of room temperature of 37 °C and relative humidity of 95 % or more, the mud was allowed to harden for a period 1.5 times the time for hardening of each cement, whereafter a slice of hardened material was obtained for examination. This slice was immersed in 50 ml of distilled water at 37 °C for 24 hours. Subsequently the water was placed in a stoppered bottle and evaporated at 150 °C until dry. The solubility was determined from the weight of the sample before immersing and the residue amount in the stoppered bottle.
   The standard for this is 2% w/w or less.
   Besides, since it is considered that, in this experiment, decomposition in water is undesirable, the decompositon percentage is determined as including both the solubility and the degree of decomposition.
4 ) Resisting force for crushing;
   A slice having a diameter of 6 mm and a height of 12 mm was prepared and stood for 24 hours under conditions of room temperature of 37 °C and relative humidity of 100 %. Thereafter, the resisting force for crushing was determined.
   The determination is carried out with a Shimazu Ograph IS-5000 and with a cross-head speed of 0.5 mm per minute.
5 ) Pathological observation with filling into a molar root canal of a grown dog;
   The molar dental pulp of a grown dog was extracted and then, without combination use of a point such as guttapercha point, each cement or kneaded mud for use as a root canal sealer was applied under pressure to fill a washed root canal. The crown part was filled and restored with the use of glass-ionomer cement. After three months, a slice for polishing which was not decalicificated was prepared by tooth extraction followed by setting with a 10 % aqueous formaline solution. After the H- E coloring treatment, a pathological observation was carried out.

Results are shown in Table 5.

As seen in Table 5, although there were no problems with examples 10 - 12, inflammation and pus blisters occurred in the examples for comparison 5 - 7.

Further, for each of the hardening materials in the examples and the examples for comparison shown above as well as in the examples for comparison 8 - 10 described below, the powder and liquid were both sterilised, mixed, and kneaded for about 1 minute, whereafter the mud obtained was allowed to hardened for an initial period equal to the time for hardening of each hardening previously determined for each hardening material, so as to make a cylindrical piece having a diameter of 4 mm and a length of 10 mm. These pieces were inserted into the femoral bone shaft of a grown dog by making a drill hole 0.2 - 0.3 mm larger than said pieces. Each hardening material was buried for 2, 4, and 6 weeks. Then, for each hardening material the slices of material which were decalcificated and not decalcificated were prepared for polishing. The slice of decalcificated material was colored with the H-E coloring and with toluidine blue to carry out a pathological observation, and the slice of material which was not decalicificated was colored with the H-E coloring for the same purpose. Furthermore, concerning the force for fixing with a bone tissue, the force required for cutting was measured, according to a pushing-out method with a cross-head speed of 0.1 mm/min. using a universal test machine of a rod cell type. Results are shown in Table 6.

### Example for comparison 8

A hardening material was used composed of a combination of a powder agent, comprising 100 % of α-TCP, with a setting solution (a liquid agent) composed of 40 % of polyacrylic acid.

### Example for comparison 9

A hardening material was used composed of a combination of a powder agent, comprising 61 % of α-TCP, 36 % of 4CP, and 3 % of HAp, with a setting solution (a liquid agent) prepared by dissolving into water polyacrylic acid and citric acid in respective proportions of 17 % and 30 %.

### Example for comparison 10

A bone cement in the PMMA series commercially available from Howmedica Co., Ltd. (trade name: Surgical Simplex), was used.

As seen in Table 6, the fixing force with a bone tissue of the listed examples was higher than that in the examples for comparison. In particular, the examples in which atelocollagen was used were better.

### ( Industrial Applicability )

The hardening material relating to the present invention can be used as a root canal sealer, as a cement and a filling agent for dental use, as bone cement and as a filling agent, and the like.

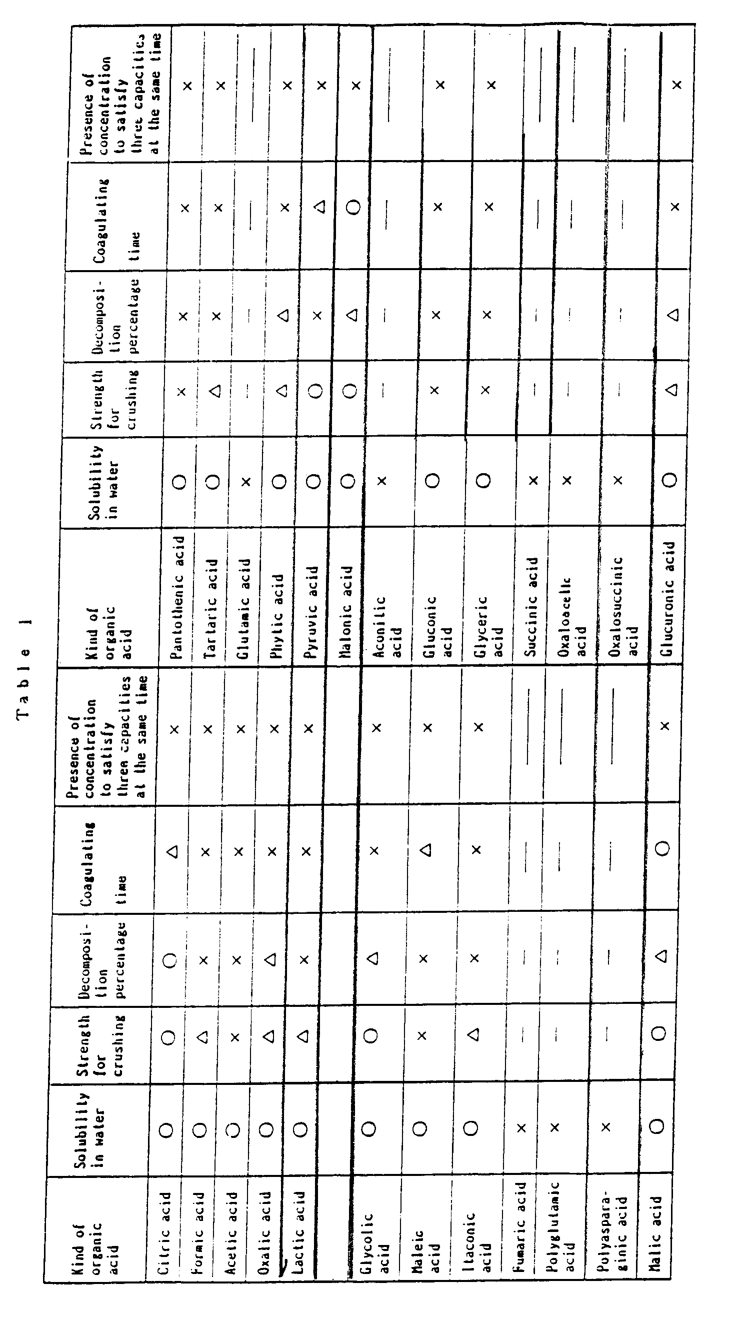

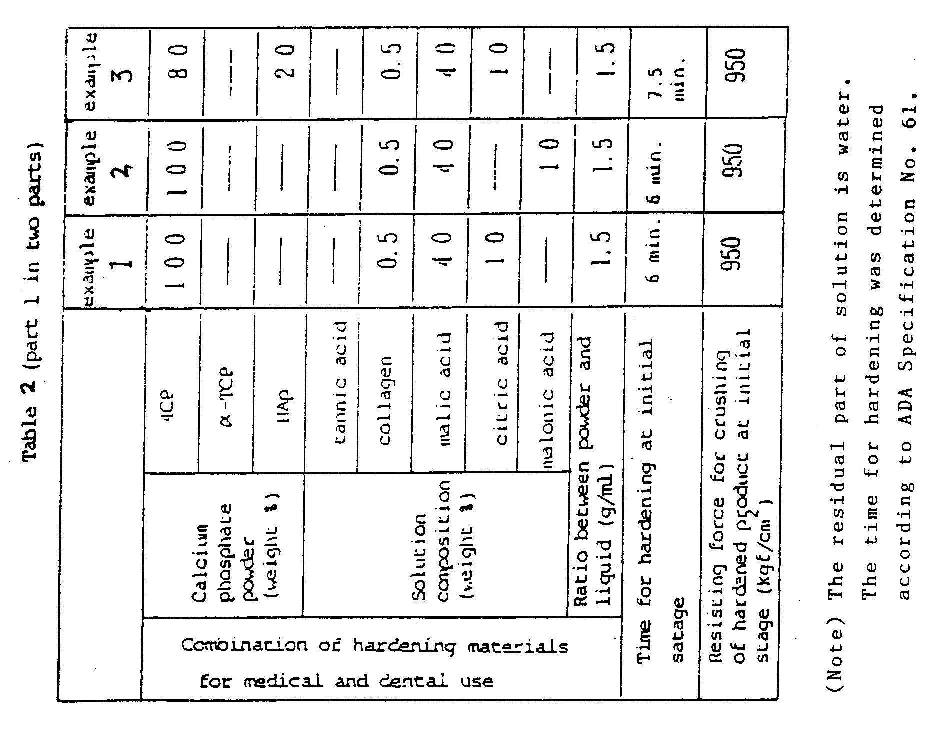

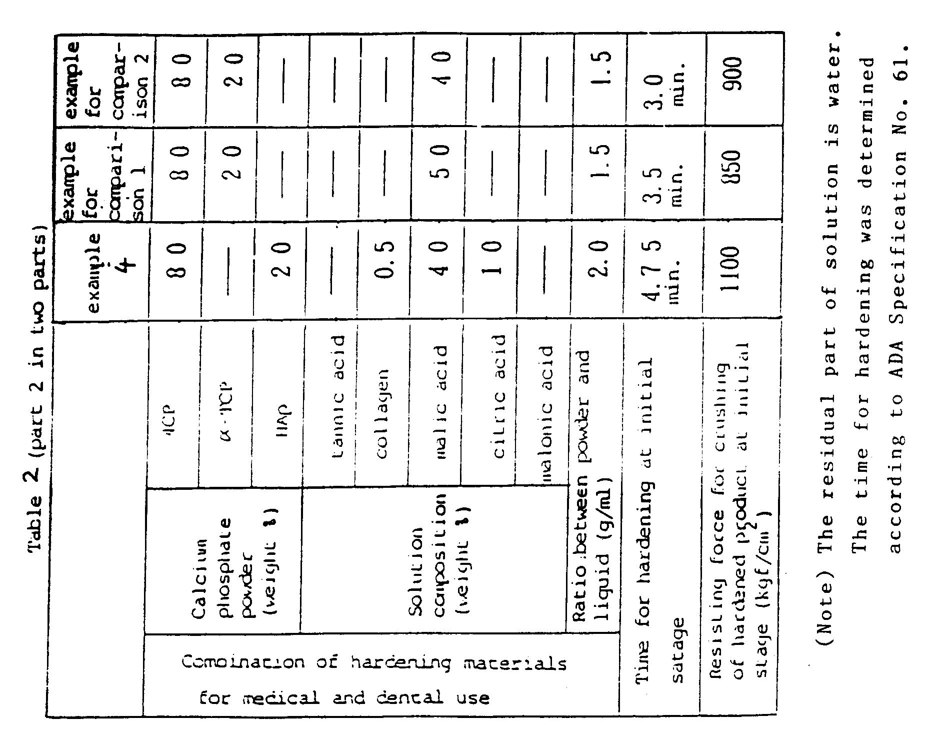

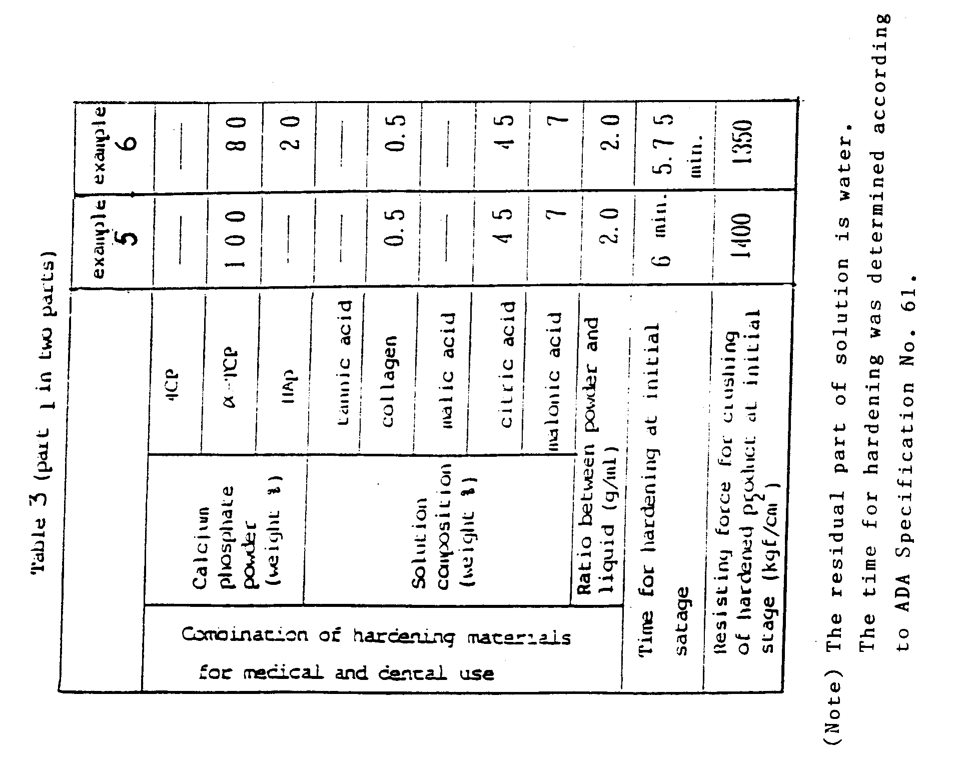

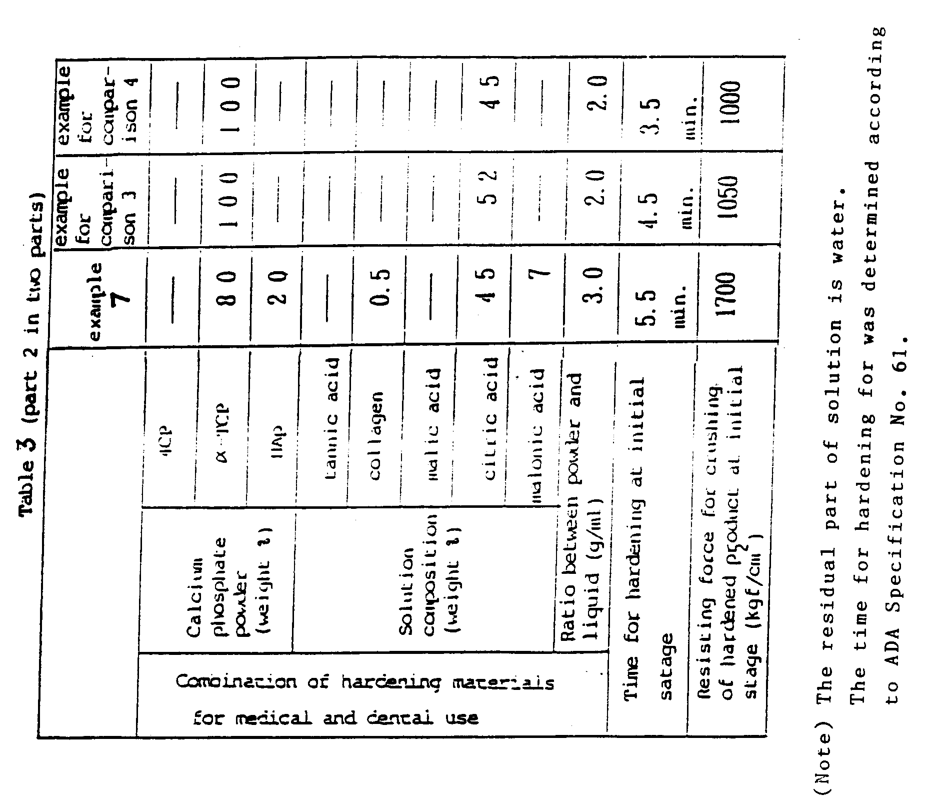

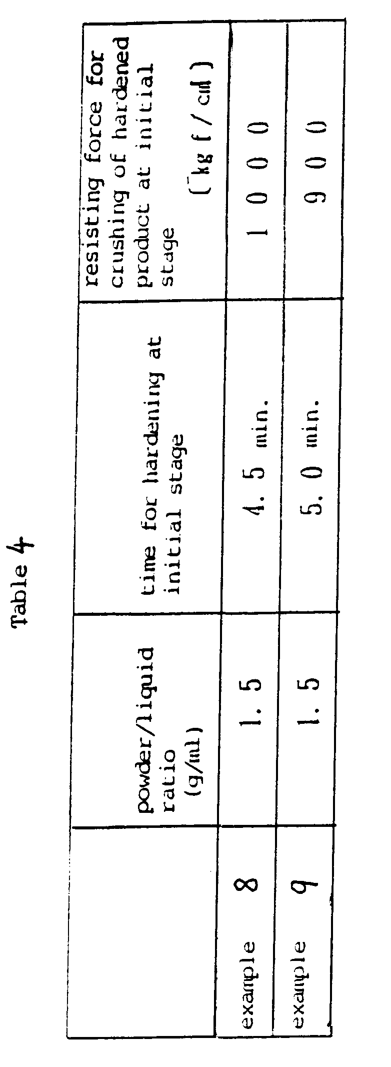

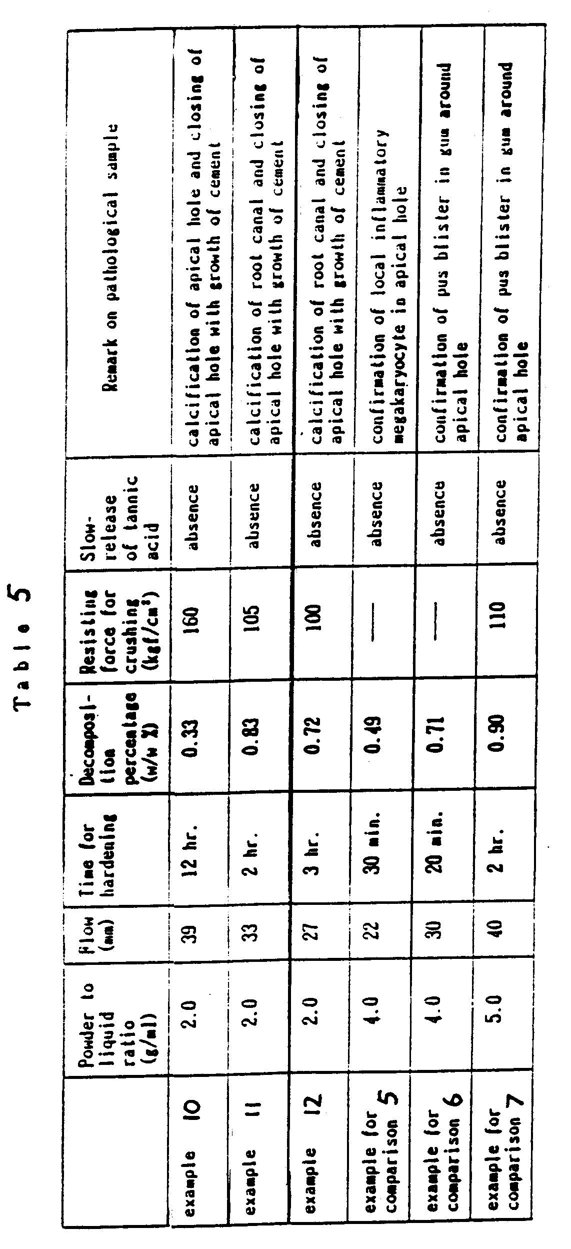

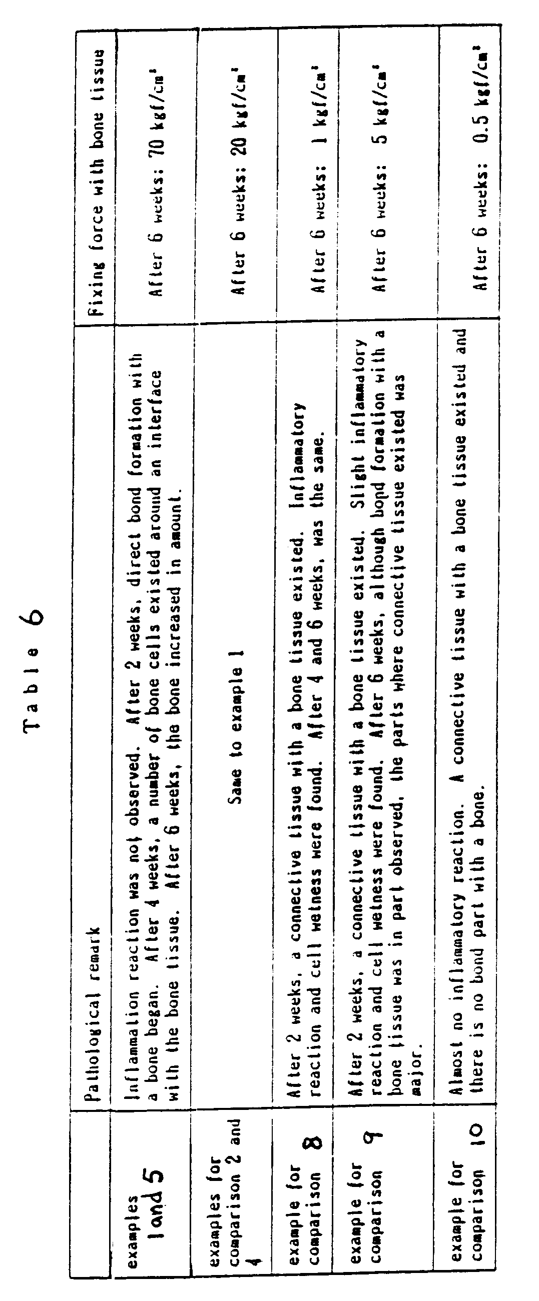

## Claims

1. A hardening material for medical and dental use, in which calcium phosphate powder containing at least either one of α-tricalcium phosphate and tetracalcium phosphate is an essential component, characterised in that at least one compound of collagen and a collagen derivative and one or more of an organic acid are used as a hardening adjuster.

2. A hardening material for medical and dental use as claimed in claim 1, in which the collagen and the collagen derivative convert into fibrils under physiological conditions.

3. A hardening material for medical and dental use as claimed in claim 2, in which the collagen and the collagen derivative require a time longer than 8 minutes for conversion into fibrils under physiological conditions.

## Patentansprüche

1. Härtungsmaterial für den medizinischen und zahnmedizinischen Gebrauch, bei welchem Kalziumphosphatpuiver, welches wenigstens α-Trikalziumphosphat oder Tetrakalziumphosphat enthält, eine wesentliche Komponente ist, dadurch gekennzeichnet, daß wenigstens eine Verbindung von Kollagen und einem Kollagenderivat und einer oder mehr als einer organischen Säure als ein Härtungseinsteller verwendet ist.

2. Härtungsmaterial für den medizinischen und zahnmedizinischen Gebrauch nach Anspruch 1, bei welchem das Kollagen und das Kollagenderivat unter physiologischen Bedingungen zu Fibrillen konvertieren.

3. Härtungsmaterial für den medizinischen und zahnmedizinischen Gebrauch nach Anspruch 2, bei welchem das Kollagen und das Kollagenderivat eine Zeit länger als acht Minuten für die Konvertierung zu Fibrillen unter physiologischen Bedingungen benötigen.

## Revendications

1. Une substance durcissante pour usage dentaire et médical, dans laquelle une poudre de phosphate de calcium contenant au moins l'un des deux composés suivants, phosphate α-tricalcique et phosphate tétracalcique, est un constituant essentiel, caractérisée en ce qu'au moins un composé du collagène et d'un dérivé du collagène et un ou plusieurs acides organiques sont utilisés comme agent pour ajuster le durcissement.

2. Une substance durcissante pour usage dentaire et médical selon la revendication 1, caractérisée en ce que le collagène et le dérivé du collagène se transforment en fibrilles dans des conditions physiologiques.

3. Une substance durcissante pour usage dentaire et médical selon la revendication 2, caractérisée en ce que le collagène et le dérivé du collagène nécessitent un temps supérieur à 8 minutes pour se transformer en fibrilles dans des conditions physiologiques.
